# EUROPEAN PATENT APPLICATION

(11) **EP 2 087 855 A2**
(43) Date of publication of application: **12.08.2009**
(21) Application number: 09001653.6
(22) Date of filing: 05.02.2009
(51) Int. Cl.: A61F 2/00

(54) **Device for the control of urinary incontinence**

(30) Priority: 07.02.2008 AR M080100541 U; 12.01.2009 MX MX09000397
(71) Applicant: Donati, Ricardo Roberto, C.A. de Buenos Aires (AR); Donati, Robert Ezequiel, C.A. de Buenos Aires (AR); Garcia, Ana Maria, Provincia de Buenos Aires (1832) (AR)
(72) Inventor: De Francesco, Juan Carlos, C.A. de Buenos Aires (1426) (AR)
(74) Representative: Arena, Giovanni

(57) **Abstract**

The device comprises a support (1) on which transverse opening (3), it is arranged the body (10a) of a penis (10) so that, its area adjacent to the uretra ductus (10b), is subject to the action of a mobile arm (5) which compressing head (6) has a prominent sector (6a) able to compress said urethra ductus (10b), exerting adjustable pressure to control urinary incontinence. In the case of the compressing head (6) with prominent (6a) and relief sectors (6b), this prominent sector (6a) is the one that compresses the area of the urethra (10b), whereas the relief sectors (6b) prevent the compression of the cavernous sectors (10a) of the penis (10), allowing their normal blood circulation. In addition, the displacement of the body (10a) of the penis (10) against the opposite area (2a) of the inner rim (2) is comfortable thanks to the presence of a wall (4) or soft surface.

## Description

### FIELD OF THE INVENTION

The present invention is related to the field of daily life and medical sciences and hygiene needs and within said field, to the external means that allow controlling urinary incontinence.

More particularly, it is relevant to a device for the control of urinary incontinence, which allows for the compression of the urethra ductus and the regulation of the intensity of the pressure exerted.

### BACKGROUND

As it is well known, there are millions of people who suffer from urinary incontinence, which seriously affects their life quality. This is the case of people who have been treated for different disorders of the urinary tract - such as prostate or bladder cancer surgery - and who, in spite of having overcome the disease, are still affected by incontinence problems.

Some ways of coping with the problem are based on the use of diapers, which result in the discomfort and limitations they generate.

Up to now, the devices known are disclosed by the US patents 2,455,859 and 2,533,924 based on compressing means with external nozzles connections.

US patent 2,756,753 shows a device with a belting or tape and a little nipper.

US patent US 3,147,754 shows a set of claws with a set of runner toothing.

US patent 3,203,421 shows a set of articulated claws with a side thread exerting uneven and sided compression.

US patent 3,926,175 shows a compressing means with magnetic control.

US patent 4,534,353 shows a device, which lacks threading, therefore, its regulation capacity is scarce.

US patents 299168, 4,942,886 and 4,800,879 show claws with one end articulated and the other end, toothed.

US patent 6,349,727 shows a device with elastic claws and an end with levers.

Finally, the device in US 4,880,016 is based on a tape with a set of openings and an anchorage.

### OBJECTS OF THE INVENTION

An object of the present invention is to allow adjustable control of urinary incontinence.

Another advantage is the high capability to adjust the pressure it exerts on the urethra ductus. This is due to the action of a compressing head driven by a mobile arm, which positional variation is based on a thread.

Another advantage is that the inclusion of prominent sectors and relief sectors in the compressing head, allows compression of the area of the urethra ductus only, keeping the penis cavernous sectors free from pressure.

Another advantage is that the incorporation of a soft wall or surface allows preventing lesions or discomfort of the penis while pressure is exerted on its body.

Another advantage is that the compressing head may be kept fixed while it turns round the mobile arm that drives it. This prevents attrition between the above mentioned head and the body of the penis.

Another advantage is that it lacks springs, toothing or any other element that might cause lesions to the user. Except the command of the mobile arm, there are no other projecting parts that may cause discomfort.

A further advantage is that its size is extremely reduced.

Other additional advantages are its constitutive simplicity and the low production cost.

### SUMMARY OF THE INVENTION

For the above purposes, the device according to the invention is characterized as indicated in the subsequent claim 1.

### BRIEF DESCRIPTION OF THE DRAWINGS

The characteristics of the invention will be better evident from the following description regarding examples of embodiment of a nonlimiting character and referring to the attached drawings, whose various figures show the following simplified views:

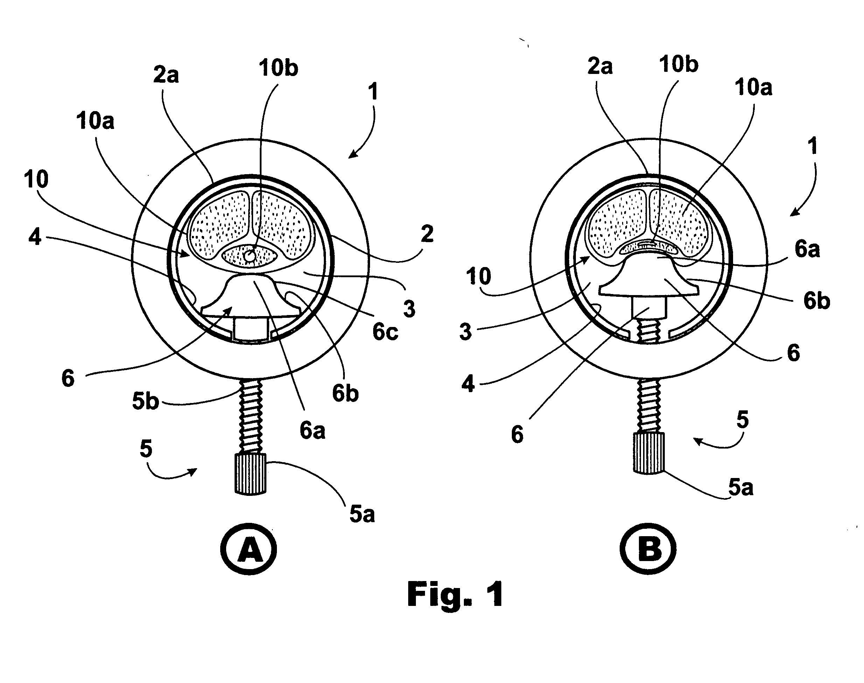

- Fig.1/A:: a front elevation view of the present device with a penis - represented in a transverse section - arranged into the transverse opening or support passage;
- Fig.1/B:: another front elevation view of the present device wherein it can be clearly appreciated how the prominent sector of the compressing head acts by closing the urethra ductus, while the relief sectors prevent the total release of the cavernous sectors of said penis.
- Fig.2/A:: a front elevation view of the present device with the mobile arm completely projected out of the transverse opening leaving it free to place the body of the penis;

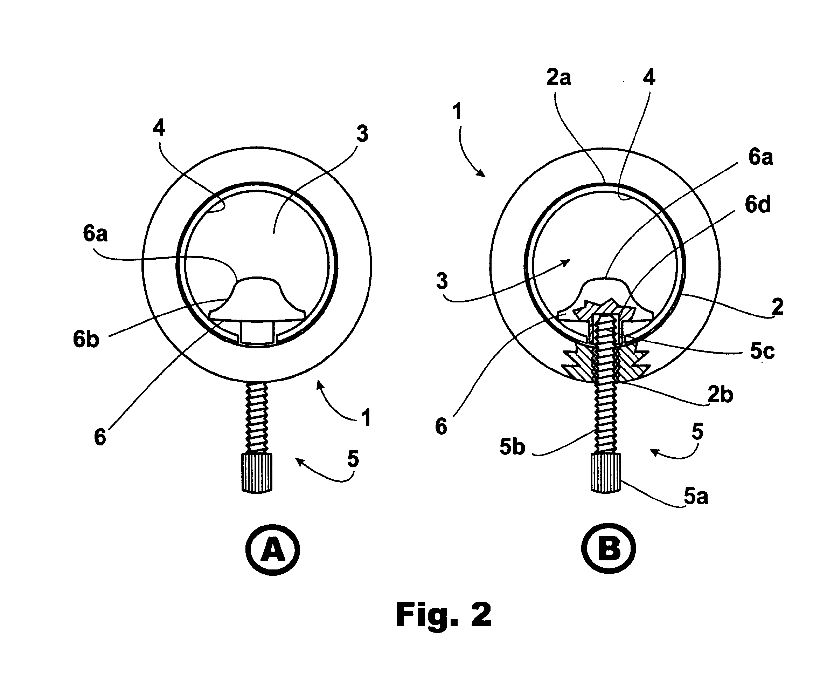

- Fig.2/B:: a partial longitudinal section, which allows appreciating the relation between the support, the mobile arm and the compressing head;

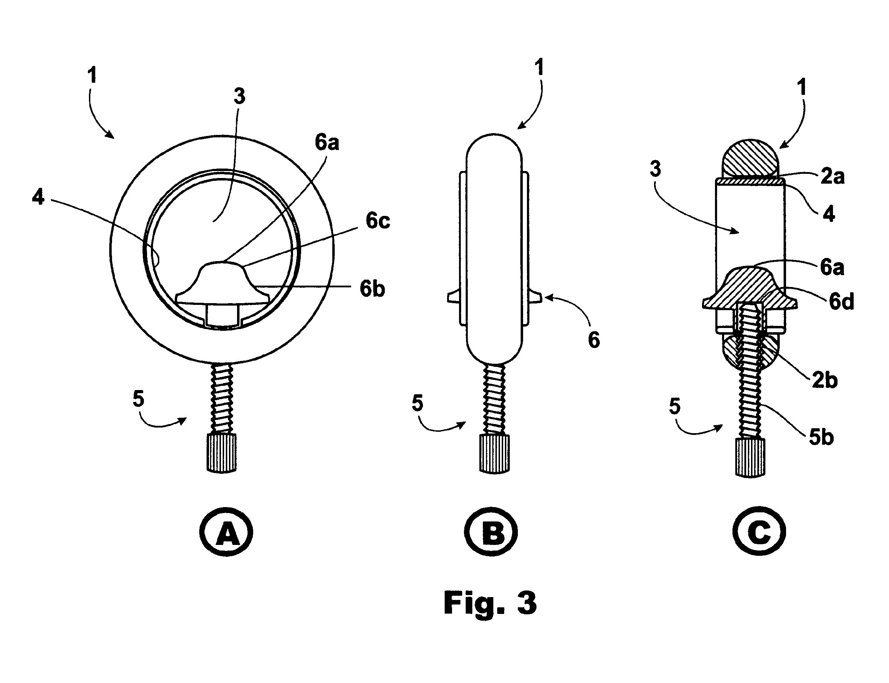

- Fig.3/A:: a front elevation view as in drawing 1A;
- Fig.3/B:: a side elevated view of the present device;
- Fig.3/C:: a transverse section, which allows appreciating the device structure in detail.

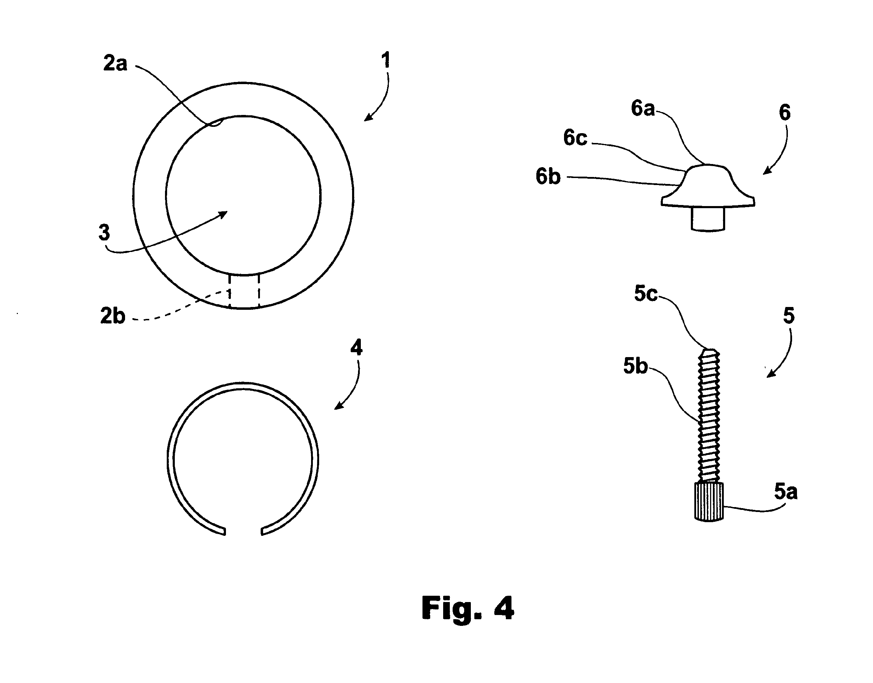

- Fig.4:: an exploded view of the present device in a side elevation view.

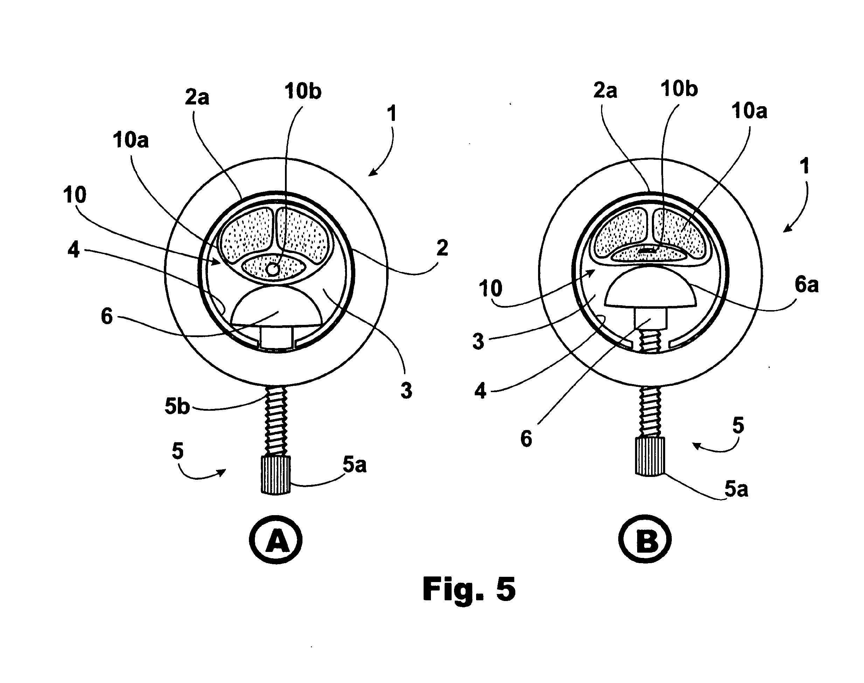

- Fig.5/A:: a front elevation view of another embodiment of the present device with a penis - represented in a transverse section - arranged into the transverse opening or support passage;
- Fig.5/B:: another front elevation view of the device of drawing A in which it can be clearly appreciated how the compressing head acts by closing the urethra ductus;

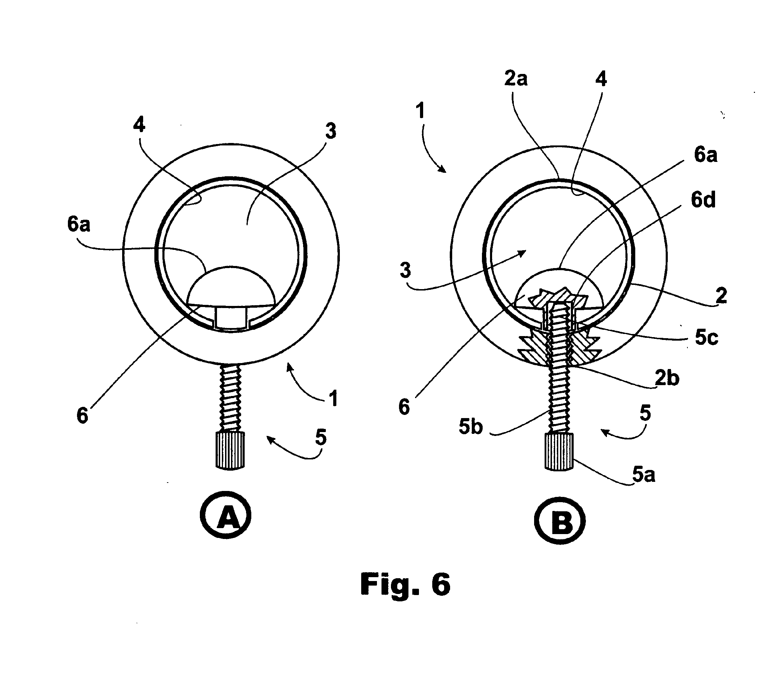

- Fig.6/A:: a front elevation view of the device in figure 5 with the mobile arm totally projected outside the transverse opening leaving it free to place the body of the penis;
- Fig.6/B:: a partial longitudinal section, which allows appreciating the relation between the support, the mobile arm and the compressing head.

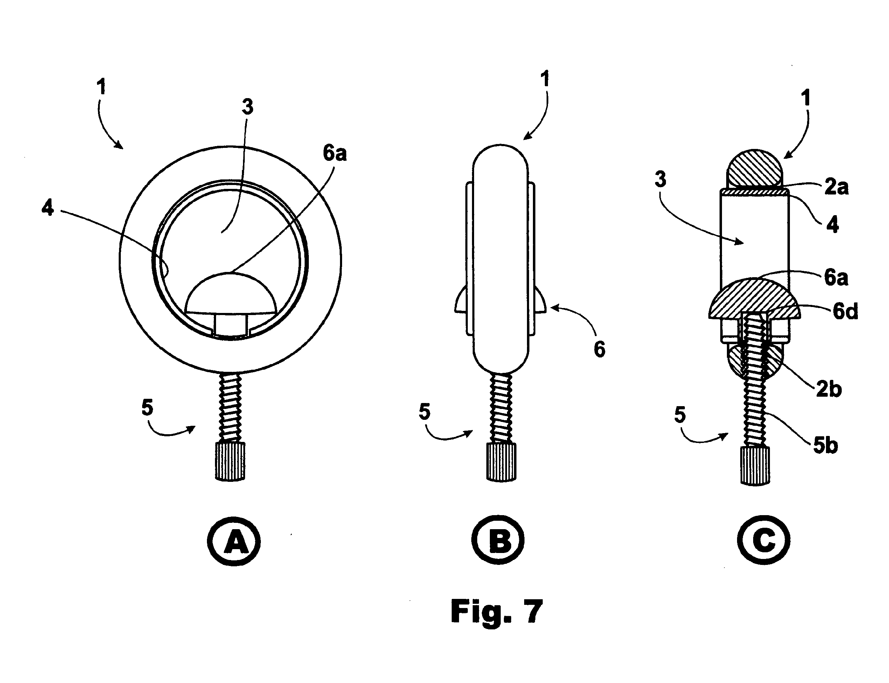

- Fig.7/A:: a front elevation view as in drawing 5A;
- Fig.7/B:: a side elevation view of the device in figure 5;
- Fig.7/C:: a transverse section, which allows appreciating the structure of the device in detail.

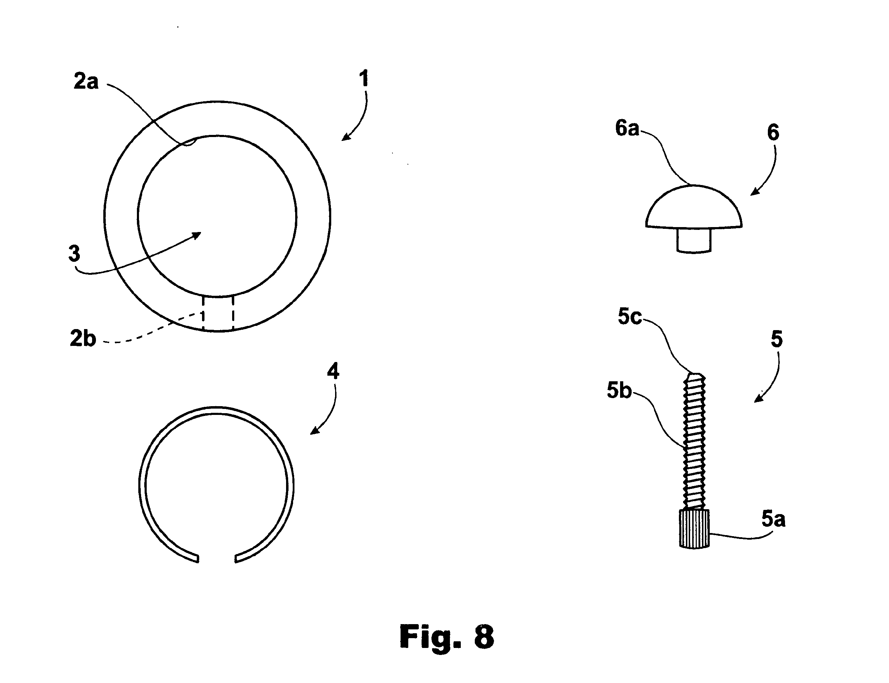

- Fig.8:: an exploded view of the device in figure 5 in a side elevation view.

### DESCRIPTION OF THE EXAMPLES OF EMBODIMENT

In the different figures, the same numbers and/or reference letters indicate equal or corresponding parts.

### List of the main references:

(1) Support.
(2) Inner rim of the support (1).
(2a) Area opposite to the compressing head (6).
(2b) Fixed thread.
(3) Transverse opening or passage for the body (10a) of the penis (10).
(4) Soft wall
(5) Compression mobile arm.
(5a) Command of the mobile arm (5).
(5b) Mobile thread.
(5c) Mobile end.
(6) Compressing head.
(6a) Prominent sector of the compressing head (6).
(6b) Relief sector.
(6c) Transition rim.
(6d) Free turn connection.
(10) Application penis.
(10a) Cavernous sectors (10).
(10b) Uretra ductus.

In general terms, the device for control of urinary incontinence according to present invention comprises a support (1) in which transverse opening (3) is arranged the body (10a) of a penis (10) so that, its area adjacent to the urethra ductus (10b), is subject to the action of a mobile arm (5) which compressing head (6) has a prominent sector (6a) able to compress said urethra ductus (10b), exerting an adjustable pressure for the control of urinary incontinency.

More particularly, the present device is applicable to the body of the penis (10) so that the closure of the urethra ductus is obtained (10b).

This device comprises a support (1) that, even though may have different shapes and configurations, in the embodiment described, forms a body mainly annular -as a fence-, which outer surface is substantially free of edges or rims susceptible to causing lesions on the user's body.

The above mentioned support (1) has some inner rims (2) which delimit a transverse opening (3) or passage able to admit the body of a penis (10) through it and receive a transverse section of the penis, so that an area adjacent to the urethra ductus (10b) is exposed to the action of the means (5)(6) of the present device.

In said transverse opening (3) or passage, a mobile arm (5) operates having positional variation means (5b) which allow its alternative advance or reverse projection on the above mentioned opening (3).

These positional variation means (5b) are given by a mobile thread (5b) which relates to a fixed thread (2b) provided by the support (1). For the displacement of said mobile arm (5), there is a command (5a) that may consist of a clamp, throttle, faceted body, lever, knob, etc.

The mobile arm (5) ends in a mobile end (5c) where it is mounted a compressing head (6), so that said compressing head (6) may have free turn capacity relative to said mobile end (5c). It has also been considered an embodiment in which the compressing head (6) has a certain capacity of axial displacement in relation to the mobile arm (5) to facilitate its adequate position.

In addition, the compressing head (6) has a surface partially or totally convex (6a) which provides higher efficiency to its compressing action on the area adjacent to the urethra ductus (10b).

In a preferred embodiment, the compressing head comprises a prominent sector (6a) -applicable to the urethra ductus (10b)- in which adjacence there are relief sectors (6b) -which prevent the compression of the cavernous bodies (10a)-. The transition between both sectors (6a)(6b) is provided by some transition rims (6c) which lack edges.

It has been provided that, at least, the area opposite (2a) to the inner rim (2) is soft so that the penis (10) can be compressed without suffering lesions.

Therefore, the inner rim (2) of the support (1) may have a soft wall (4), cover or surface that covers it totally or partially. Operation: The transverse opening (3) or passage allows that through the opening (3) the application penis (10) may be placed, until being arranged in such a way that a sector adjacent to the urethra ductus (10b) is subject to the action of the control means (5)(6).

In these conditions, by means of the respective command (5a), the mobile arm is driven (5) so that its mobile thread (5b) moves through the fixed thread (2b) of the support (1). This allows the advance of the compressing head (6) until its wall or convex surface (6a) contacts the area adjacent to the urethra (10b), from there onwards, being able to adjust the pressure exerted by the above mentioned head (6).

Once the contact is made, the compressing head (6) may stop turning although the mobile arm (5) continues turning due to the free turn connection (6d), which allows preventing any attrition with the body (10a) of the penis (10).

In the case of the compressing head (6) with prominent (6a) and relief (6b) sectors, this prominent sector (6a) is the one that compresses the area of the urethra (10b), whereas the relief sectors (6b) prevent the compression of the cavernous sectors (10a) of the penis (10), allowing their normal blood circulation.

In addition, the movement of the body (10a) of the penis (10) against the opposite area (2a) of the inner rim (2) is comfortable, thanks to the presence of a soft wall (4) or surface.

It has also been provided that the outer surface of the support (1) is free of edges or rims that might affect areas adjacent to the user¦ s body.

Finally, it has been provided the possibility that the device has a set of two or more interchangeable compressing heads (6) with different shapes and for different uses, anatomies, times, etc.

While the invention has been described with reference to few particular examples of embodiment, it is evident that numerous modifications, variations and substitutions of elements with others of a functionally equivalent type may be applied without thereby abandoning the spirit of the invention and the scope of the following claims.

## Claims

1. Device for the control of urinary incontinence, applicable around the body of a penis having the capacity to compress selectively the area adjacent to the urethra ductus, **characterized in that** it comprises:
- a support (1) provided with a transverse opening (3) able to admit the body (10a) of the penis (10) through it,
- a mobile arm (5) of adjustable compression on said opening (3), with adjustable compression works,
where said mobile arm (5) comprises a command means (5a), positional variation means (5b) and a compressing head (6) of the area adjacent to the urethra ductus (10b) of the penis arranged to that purpose, and where on said support (1), at least, the inner area (2a) opposite to the mobile arm (5) is soft.

2. Device for the control of urinary incontinence, according to claim 1, **characterized in that** the support (1) consists of a fence that limits a passage able to receive the penis through it, said fence having a compressing mobile arm, which, through said passage, acts, on the area where the urethra ductus of the application penis is arranged.

3. Device for the control of urinary incontinence, according claim 1, **characterized in that** the support (1) comprises an annular fence.

4. Device for the control of urinary incontinence, according claim 1, **characterized in that** the transverse opening (3) is a passage able to allow the entry of a penis through it, until the area adjacent to the urethra of said penis is exposed to the action of the compressing mobile arm.

5. Device for the control of urinary incontinence, according claim 1, **characterized in that** the positional variation means (5b) of the compressing mobile arm are provided by a thread.

6. Device for the control of urinary incontinence, according claim 1, **characterized in that** the compressing head (6) of the mobile arm is at least, partially convex.

7. Device for the control of urinary incontinence, according claim 1, **characterized in that** the compressing head (6) of the mobile arm is prominent in the sector that compresses the urethra ductus (10b) of the application penis.

8. Device for the control of urinary incontinence, according claim 1, **characterized in that** the compressing head (6) of the mobile arm comprises a prominent sector (6a), able to compress the urethra ductus of the application penis, and adjacent relief sectors (6b) which prevent the compression of the cavernous bodies of said penis.

9. Device for the control of urinary incontinence, according claim 1, **characterized in that** the compressing head (6) has free turn capacity respect the mobile arm (5) at which end it is mounted.

10. Device for the control of urinary incontinence, according claim 1, **characterized in that** the compressing head (6) has a certain capacity of axial displacement respect the mobile arm.

11. Device for the control of urinary incontinence, according claim 1, **characterized in that** the command means (5a) comprises an arm.

12. Device for the control of urinary incontinence, according claim 1, **characterized in that,** at least, the inner area (2a) of the support (1) opposite the mobile arm is soft.

13. Device for the control of urinary incontinence, according claim 1, **characterized in that** in the support (1), at least, the inner area opposite the mobile arm (5) comprises a soft cover.

14. Device for the control of urinary incontinence, according claim 1, **characterized in that** the body of the support (1) forms an outer surface substantially free of edges.

15. Device for the control of urinary incontinence, according claim 1, **characterized in that** it comprises a set of interchangeable compressing heads.
